# EUROPEAN PATENT APPLICATION

(11) **EP 4 599 859 A1**
(43) Date of publication of application: **13.08.2025**
(21) Application number: 23874073.2
(22) Date of filing: 27.06.2023
(51) Int. Cl.: A61M 1/06

(54) **MUFFLING AIR PUMP STRUCTURE FOR BREAST PUMP**

(30) Priority: 08.10.2022 CN 202222647287 U
(71) Applicant: Guangdong Youju Advanced New Materials Co., Ltd., Jiangmen, Guangdong 529040 (CN); Guangdong Golden Baby Health Supplies Co., Ltd., Jiangmen, Guangdong 529040 (CN)
(72) Inventor: NI, Jinxiang, Jiangmen, Guangdong 529040 (CN); WANG, Xianwen, Jiangmen, Guangdong 529040 (CN); LI, Haoyuan, Jiangmen, Guangdong 529040 (CN)
(74) Representative: Elzaburu S.L.P.
(86) International application number: PCT/CN2023/102832
(87) International publication number: WO 2024/074058

(57) **Abstract**

A muffling air pump structure for a breast pump is provided. A bracket is hermeticallyprovided at a housing having an open end. An outer side of the bracket is provided with a motor. A shaft of the motor is a screw, penetrates through the bracket, extends into the housing, and is connected to a piston through a screw nut. The bracket is provided with a muffling airway that communicates an inner side with the outer side and is configured to reduce a volume of noise. The muffling airway includes a plurality of muffling chambers that are sequentially connected. Each of the muffling chambers is provided with a muffling hole. Some of the muffling chambers are provided with muffling plates. The muffling plate further divides the muffling chamber into a large chamber and a smaller chamber, such that a multi-stage muffling structure is formed, significantly reducing the volume of the noise. The muffling air pump structure greatly reduces the working noise of the breast pump, thereby avoiding excessive environmental disturbance, ensuring a good resting environment for the newborn and the nursing mother, and improving the comfort of using the breast pump.

## Description

### TECHNICAL FIELD

The present utility model relates to a breast pump, and in particular to a muffling air pump structure in a main unit of a breast pump.

### BACKGROUNDTECHNOLOGY

Breastpumps are designed to extract milk from breasts, and are categorized into manual and electric types. As a critical component of the electric breast pump, the air pump is configured to control air pressure. Prior air pumps typically include direct current motor (DCM)-driven diaphragm air pumps and gear-reduction DCM-driven piston pumps. In these air pumps, the piston of the air cylinder is connected to the gear set and the speed changer. The operation of the air pump will generate significant noise that severely impacts the usage environment. Especially for newborns and their mothers, excessive noise will severely impact newborns' sleep and growth, and exacerbate emotional stress in nursing mothers, whose emotional well-being is already vulnerable during the postnatal period, substantially increasing the risk of postpartum depression in nursing mothers.

### CONTENT OF THE UTILITY MODEL

An objective of the present utility model is to provide a muffling air pump structure with low working noise for a breast pump, which reduces the noise in the usage environment, thereby improving the comfort of newborns and their mothers.

In the present utility model, the muffling air pump structure for a breast pump includes a housing that is cylindrical and has an open end, where a bracket is hermeticallyprovided at the open end of the housing; a central part of an inner side of the bracket extends into the housing to form a hollow mounting post; an outer side of the bracket is connected to a motor; the bracket is provided with a muffling airway that communicates the inner side with the outer side and is configured to reduce a volume of noise; a shaft of the motor penetrates through the bracket and the mounting post of the bracket, and is connected to a piston; and the outer side of the bracket is provided with a hood configured to cover the motor.

Furthermore, the muffling airway includes a hollow airway provided inside the bracket; the inner side and the outer side of the bracket are respectively provided with an airway inlet and an airway outlet that are communicated with the airway; the airway from the airway inlet to the airway outlet is internally divided into a plurality of muffling chambers that are sequentially connected; and each of walls of all the muffling chambers facing the airway inlet is provided with a muffling hole that is communicated with the airway inlet or a previous muffling chamber.

Furthermore, the bracket includes a bracket body; a central part of an inner side of the bracket body extends into the housing to form the mounting post; the inner side of the bracket body is recessed inward at a peripheral position of the mounting post and forms an internal space of the muffling chambers; and a cover plate is provided to cover the internal space of the muffling chambers.

Furthermore, the muffling hole is located at a radial inner/outer end of the wall of the muffling chamber, and the muffling hole faces a radial outer/inner wall of the muffling chamber.

Furthermore, the muffling chambers include an inlet muffling chamber connected to the airway inlet, an outlet muffling chamber connected to the airway outlet, and an intermediate muffling chamber between the inlet muffling chamber and the outlet muffling chamber; each of the walls of all the muffling chambers adjacent to the airway inlet is"<"-shaped; an upper end and a lower end of the "<"-shaped wall are respectively connected to the radial inner and outer walls of the muffling chamber; the muffling hole is located at an upper part of the "<"-shaped wall and faces the outer wall of the muffling chamber; as for the inlet muffling chamber and the intermediate muffling chamber, a midpoint position of the "<"-shaped wall facing away from the airway inlet is provided with a muffling plate extending towards the inner wall of the muffling chamber; and a gap is formed between the muffling plate and the inner wall of the muffling chamber.

Furthermore, a width of the gap is not greater than one tenth of a width of the muffling chamber.

Furthermore, the airway outlet is located at a bottom of the outlet muffling chamber adjacent to the outer wall; and the wall of the outlet muffling chamber facing the airway inlet is provided with a partition extending towards another wall.

Furthermore, the motor is a stepper motor.

Furthermore, the shaft is a screw and is connected to the piston through a screw nut.

In the muffling air pump structure for a breast pump in the present utility model, main sources of the noise in an air pump include the rotation of the shaft of the motor, the relative movement between the shaft and the piston, the friction between the piston and an inner wall of the housing, and an airflow caused by a change in an air pressure inside the housing. These sources of the noise are all enclosed within the housing and the bracket to avoid direct discharge to the external environment. An interior of the housing is divided into two chambers by the piston. One chamber of the housing facing away from the motor is provided with an air tube joint and connected to a suction bottle through an air tube, thereby changing an air pressure of the suction bottle for milk suction. The other chamber forms a positive pressure when the piston moves towards the motor, causing the air inside the chamber to be discharged to the external environment through the muffling airway. After the air passes through the muffling airway, the volume of the noise is reduced by the muffling airway, effectively reducing the working noise of the breast pump. The muffling air pump structure greatly reduces the working noise of the breast pump, thereby avoiding excessive environmental disturbance, ensuring a good resting environment for the newborn and the nursing mother, and improving the comfort of using the breast pump.

### DESCRIPTION OF THE DRAWINGS

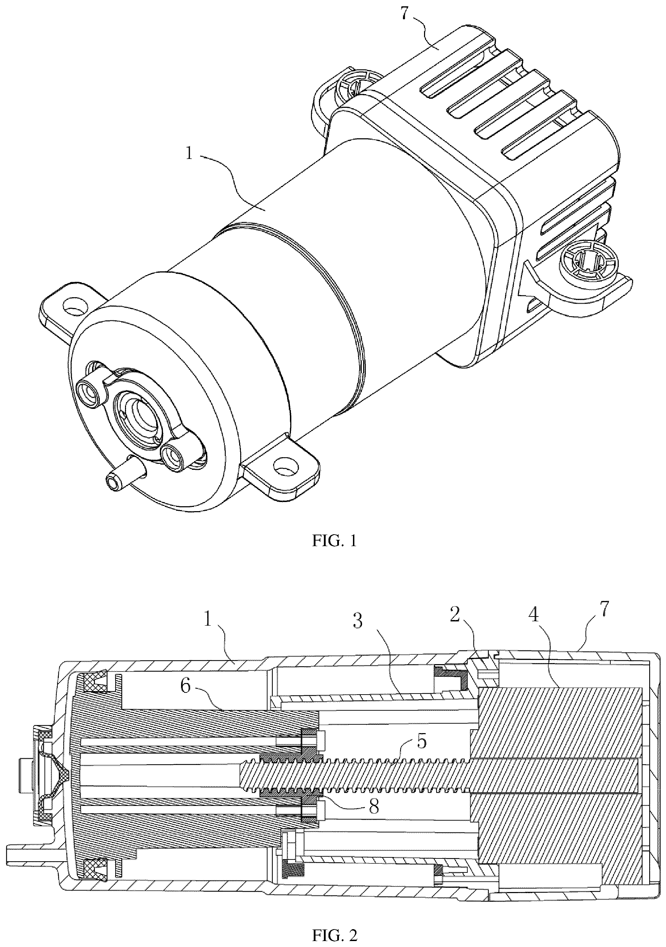

FIG. 1 is a structural schematic diagram of a muffling air pump structure for a breast pump;
FIG. 2 is a sectional structural schematic diagram of the muffling air pump structure for a breast pump;
FIG. 3 is a structural schematic diagram of a bracket;
FIG. 4 is a structural schematic diagram of the bracket and a muffling airway provided inside the bracket;
FIG. 5 is a partial structural schematic diagram of the muffling airway shown in FIG. 4; and
FIG. 6 is a top-view structural schematic diagram of the bracket and the muffling airway provided inside the bracket.

### SPECIFIC IMPLEMENTATIONS

The following clearly and completely describes the technical solutions in the embodiments of the present utility model with reference to drawings. Apparently, the described embodiments are merely some rather than all of the embodiments of the present utility model. All other embodiments obtained by those of ordinary skill in the art based on the embodiments of the present utility model without creative efforts shall fall within the protection scope of the present utility model.

It should be noted that all the directional indications (such as upper, lower, left, right, front, back, top, bottom, inner, outer, vertical, transverse, longitudinal, anticlockwise, clockwise, circumferential, radial, and axial) in the embodiments of the present utility model are merely used to explain a relative position relationship or motion situations of the components in a specific gesture (as shown in the figures). If the specific gesture changes, the directivity indication also changes accordingly.

Moreover, the terms such as "first" and "second" described in the embodiments of the present utility model are used only for the purpose of description and are not intended to indicate or imply relative importance, or implicitly indicate the number of the indicated technical features. Therefore, features defined by "first" and "second" may explicitly or implicitly include at least one of the features. Further, the technical solutions of the various embodiments may be combined together on the basis that the combination is implementable by those of ordinary skill in the art. In case a combination of the technical solutions is contradictory or infeasible, such a combination is deemed inexistent and not falling within the protection scope of the present utility model.

The present utility model proposes a muffling air pump structure for a breast pump.

In an embodiment of the present utility model, the muffling air pump structure for a breast pump includes housing 1 that is cylindrical and has an open end. Bracket 2 is hermeticallyprovided at the open end of the housing. A central part of an inner side of the bracket extends into the housing to form hollow mounting post 3. An outer side of the bracket is connected to motor 4. The bracket is provided with a muffling airway that communicates the inner side with the outer side and is configured to reduce a volume of noise. Shaft 5 of the motor penetrates through the bracket and the mounting post of the bracket, and is connected to piston 6. The outer side of the bracket is provided with hood 7 configured to cover the motor.

In the muffling air pump structure for a breast pump, main sources of the noise in an air pump include the rotation of the shaft of the motor, the relative movement between the shaft and the piston, the friction between the piston and an inner wall of the housing, and an airflow caused by a change in an air pressure inside the housing. These sources of the noise are all enclosed within the housing and the bracket to avoid direct discharge to the external environment. An interior of the housing is divided into two chambers by the piston. One chamber of the housing facing away from the motor is provided with an air tube joint and connected to a suction bottle through an air tube, thereby changing an air pressure of the suction bottle for milk suction. The other chamber forms a positive pressure when the piston moves towards the motor, causing the air inside the chamber to be discharged to the external environment through the muffling airway. After the air passes through the muffling airway, the volume of the noise is reduced by the muffling airway, effectively reducing the working noise of the breast pump.

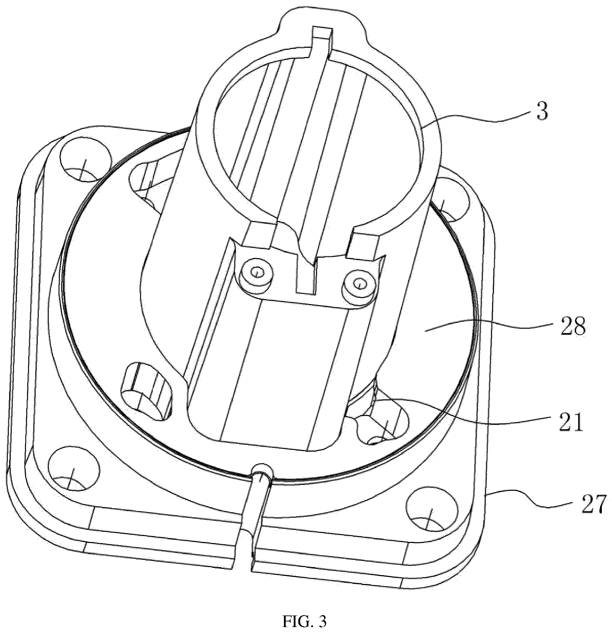

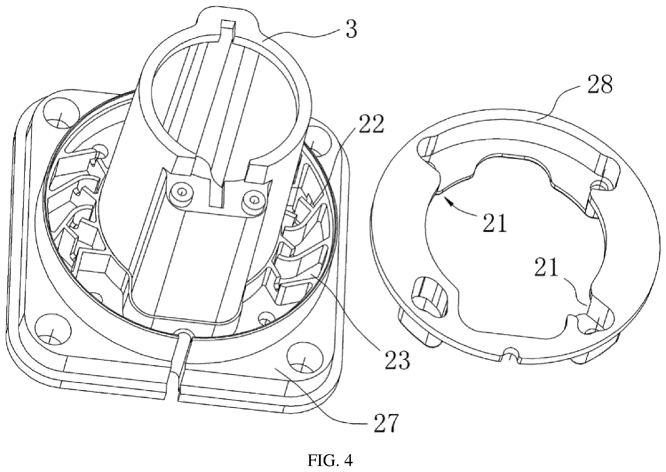

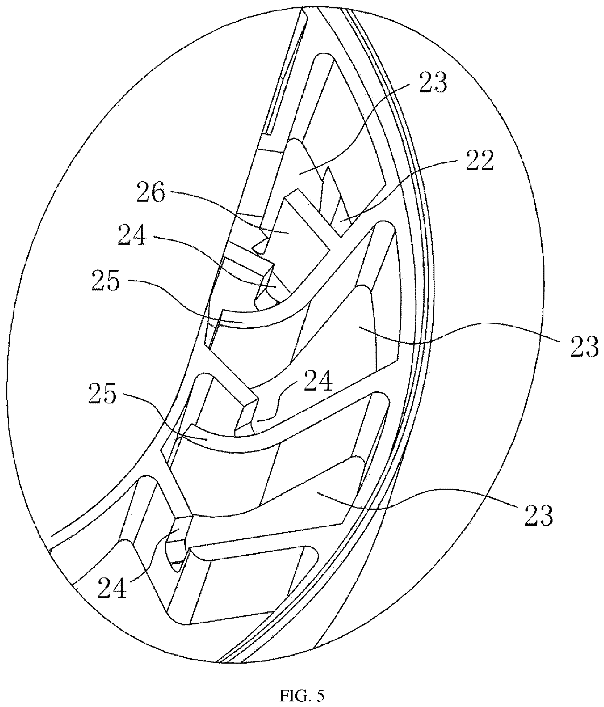

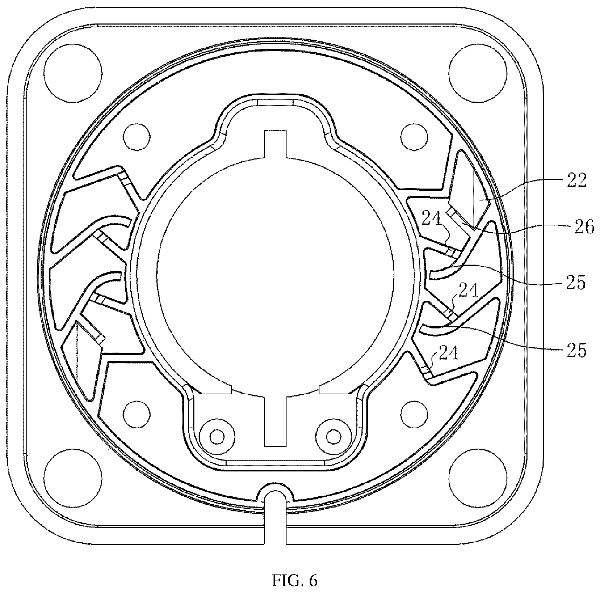

In the muffling air pump structure for a breast pump of the breast pump, the muffling airway includes a hollow airway provided inside the bracket 2. The inner side and the outer side of the bracket 2 are respectively provided with airway inlet 21 and airway outlet 22 that are communicated with the airway. The airway from the airway inlet to the airway outlet is internally divided into a plurality of muffling chambers 23 that are sequentially connected. Each of walls of all the muffling chambers facing the airway inlet is provided with muffling hole 24 that is communicated with the airway inlet or a previous muffling chamber. Thus, through the setting of the muffling chambers, a sound wave undergoes multiple reflections and interferences within the muffling chambers, thereby weakening the sound wave entering the next muffling chamber. Due to the multi-stage muffling roles of the plurality of muffling chambers, the muffling effect is greatly improved and the working noise is effectively reduced. In addition, the muffling hole 24 can be located at a radial inner/outer end of a wall of the muffling chamber 23, and the muffling hole faces a radial outer/inner wall of the muffling chamber. In this way, the sound wave must undergo more reflections and interferences before entering the next muffling chamber, further weakening the intensity of the sound wave. In addition, the muffling chambers 23 include an inlet muffling chamber connected to the airway inlet 21, an outlet muffling chamber connected to the airway outlet 22, and an intermediate muffling chamber between the inlet muffling chamber and the outlet muffling chamber. As shown in FIGS. 3 to 6, each of the walls of all the muffling chambers adjacent to the airway inlet is"< "-shaped. An upper end and a lower end ofthe "<"-shaped wall are respectively connected to the radial inner and outer walls of the muffling chamber. The muffling hole 24 is located at an upper part of the "<"-shaped wall and faces the outer wall of the muffling chamber. As for the inlet muffling chamber and the intermediate muffling chamber, a midpoint position of the "<"-shaped wall facing away from the airway inlet is provided with muffling plate 25 extending towards the inner wall of the muffling chamber. A gap is formed between the muffling plate and the inner wall of the muffling chamber, and a width of the gap is not greater than one tenth of a width of the muffling chamber. In this way, the muffling chamber can be further divided into two chambers, a large chamber and a small chamber. The large chamber has an outer width greater than an inner width thereof. The sound wave is directed from the muffling hole towards the outer side and then reflected towards the inner side, causing multiple reflections and mutual reflections, thereby weakening the sound wave multiple times. The weakened sound wave enters the small chamber through the gap at the muffling plate, and then is directed towards the next muffling chamber. Thus, a multi-stage muffling structure is formed, which greatly reduces the volume of the noise. Meanwhile, the airway outlet 23 is located at a bottom of the outlet muffling chamber adjacent to the outer wall. The wall of the outlet muffling chamber facing the airway inlet 21 is provided with partition 26 extending towards another wall. The design also improves the noise reduction capability of the outlet muffling chamber. This muffling airway structure effectively reduces the working noise of the breast pump, thereby improving the comfort of use.

In the muffling air pump structure for a breast pump, the bracket 2 includes bracket body 27. A central part of an inner side of the bracket body extends into the housing 1 to form the mounting post 3. The inner side of the bracket body is recessed inward at a peripheral position of the mounting post and forms an internal space of the muffling chambers 23. Cover plate 28 is provided to cover the internal space of the muffling chambers. Thus, the bracket body and the cover plate together form the muffling airway. That is to say, the bracket is set as a split type, which improves the convenience of connection between components and manufacturing of the bracket and the muffling airway in the bracket.

In the muffling air pump structure for a breast pump, the motor 4 is a stepper motor, and the shaft 5 is a screw and is connected to the piston 6 through screw nut 8. The use of the stepper motor and the screw nut pair improves working accuracy, effectively reduces working noise, and further enhance user comfort.

The above described are merely preferred embodiments of the present utility model, which are not intended to limit the scope of the patent of the present utility model. Any equivalent structure transformation made based on the description and drawings of the present utility model, or direct or indirect application thereof in other related technical fields, should fall within the protection scope of the patent of the present utility model.

## Claims

1. A muffling air pump structure for a breast pump, comprising a housing (1) that is cylindrical and has an open end, wherein a bracket(2) is hermeticallyprovided at the open end of the housing; a central part of an inner side of the bracket extends into the housing to form a hollow mounting post (3); an outer side of the bracket is connected to a motor (4); the bracket is provided with a muffling airway that communicates the inner side with the outer side and is configured to reduce a volume of noise; a shaft (5) of the motor penetrates through the bracket and the mounting post of the bracket, and is connected to a piston (6); and the outer side of the bracket is provided with a hood (7) configured to cover the motor.

2. The muffling air pump structure for the breast pump according to claim 1, wherein the muffling airway comprises a hollow airway provided inside the bracket (2); the inner side and the outer side of the bracket (2) are respectively provided with an airway inlet (21) and an airway outlet (22) that are communicated with the airway; the airway from the airway inlet to the airway outlet is internally divided into a plurality of muffling chambers (23) that are sequentially connected; and each of walls of all the muffling chambers facing the airway inlet is provided with a muffling hole (24) that is communicated with the airway inlet or a previous muffling chamber.

3. The muffling air pump structure for the breast pump according to claim 2, wherein the bracket (2) comprises a bracket body (27); a central part of an inner side of the bracket body extends into the housing (1) to form the mounting post (3); the inner side of the bracket body is recessed inward at a peripheral position of the mounting post and forms an internal space of the muffling chambers (23); and a cover plate (28) is provided to cover the internal space of the muffling chambers.

4. The muffling air pump structure for the breast pump according to claim 2, wherein the muffling hole (24) is located at a radial inner/outer end of the wall of the muffling chamber (23), and the muffling hole faces a radial outer/inner wall of the muffling chamber.

5. The muffling air pump structure for the breast pump according to claim 2, 3 or 4, wherein the muffling chambers (23) comprise an inlet muffling chamber connected to the airway inlet (21), an outlet muffling chamber connected to the airway outlet (22), and an intermediate muffling chamber between the inlet muffling chamber and the outlet muffling chamber; each of the walls of all the muffling chambers adjacent to the airway inlet is"<"-shaped; an upper end and a lower end of the "<"-shaped wall are respectively connected to the radial inner and outer walls of the muffling chamber; the muffling hole (24) is located at an upper part of the "<"-shaped wall and faces the outer wall of the muffling chamber; as for the inlet muffling chamber and the intermediate muffling chamber, a midpoint position of the "<"-shaped wall facing away from the airway inlet is provided with a muffling plate (25) extending towards the inner wall of the muffling chamber; and a gap is formed between the muffling plate and the inner wall of the muffling chamber.

6. The muffling air pump structure for the breast pump according to claim 5, wherein a width of the gap is not greater than one tenth of a width of the muffling chamber.

7. The muffling air pump structure for the breast pump according to claim 5, wherein the airway outlet (23) is located at a bottom of the outlet muffling chamber adjacent to the outer wall; and the wall of the outlet muffling chamber facing the airway inlet (21) is provided with a partition (26) extending towards another wall.

8. The muffling air pump structure for the breast pump according to claim 1, wherein the motor (4) is a stepper motor.

9. The muffling air pump structure for the breast pump according to claim 8, wherein the shaft (5) is a screw and is connected to the piston (6) through a screw nut (8).
